(19) Europäisches Patentamt / European Patent Office / Office européen des brevets

(11) **EP 3 470 836 B1**

(12) **FASCICULE DE BREVET EUROPEEN**

(45) Date de publication et mention de la délivrance du brevet:
**22.04.2020 Bulletin 2020/17**

(51) Int Cl.:
***G01N 33/34*** *(2006.01)* ***D21H 27/00*** *(2006.01)*

(21) Numéro de dépôt: **18198542.5**

(22) Date de dépôt: **04.10.2018**

(54) **APPAREIL DE MESURE DE LA CAPACITÉ D'ABSORPTION D'UN PAPIER ABSORBANT**

GERÄT ZUM MESSEN DER ABSORPTIONSFÄHIGKEIT EINES ABSORBIERENDEN PAPIERS

DEVICE FOR MEASURING THE ABSORPTION CAPACITY OF ABSORBENT PAPER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.10.2017 FR 1759490**
**24.01.2018 FR 1850561**

(43) Date de publication de la demande:
**17.04.2019 Bulletin 2019/16**

(73) Titulaire: **Narvil**
**38320 Eybens (FR)**

(72) Inventeurs:
- **LIVRAN, Jean-Pierre**
  **38000 GRENOBLE (FR)**
- **LIVRAN, Damien**
  **38660 LUMBIN (FR)**
- **LIVRAN, Julien**
  **38410 SAINT MARTIN D'URIAGE (FR)**
- **CORREARD, Ambre**
  **38570 GONCELIN (FR)**

(74) Mandataire: **Cabinet Beaumont**
**4, Place Robert Schuman**
**B.P. 1529**
**38025 Grenoble Cedex 1 (FR)**

(56) Documents cités:
- **Christopher Stieger: "Tissue Water Absorption Tester", , 6 février 2015 (2015-02-06), XP055501096, Extrait de l'Internet: URL:https://xell.at/wp-content/uploads/2015/03/Data-Sheet-13.100-Tissue-Absorption-Tester.pdf [extrait le 2018-08-21]**
- **Xell: "Xell Tissue Absorption Tester Digital Model", YouTube, 11 mai 2017 (2017-05-11), pages 1-1, XP054978609, Extrait de l'Internet: URL:https://www.youtube.com/watch?v=ryqsJbr-OM0 [extrait le 2018-08-22]**
- **Anonymous: "Tissue Absorption Tester", , 18 juillet 2017 (2017-07-18), XP055501104, Extrait de l'Internet: URL:https://www.rycobel.com/assets/uploads/downloads/water-absorptie-tester/RL-TAT-A_171127_134422.pdf [extrait le 2018-08-21]**
- **N Giede: "TISSUE ABSORPTION TESTER", , décembre 2014 (2014-12), XP055501111, Extrait de l'Internet: URL:http://www.mctec.nl/wp-content/uploads/2014/12/Water-Absorption-Tester.pdf [extrait le 2018-08-21]**

EP 3 470 836 B1

## Description

Domaine

La présente demande concerne un appareil de mesure du temps d'immersion d'un papier "tissue" et de sa capacité d'absorption d'eau.

Exposé de l'art antérieur

Les papiers absorbants, et plus particulièrement les papiers "tissue", tels que le papier mouchoir, le papier linge, le papier hygiénique et les couches, sont des papiers fabriqués par des machines à papier spécifiques et destinés à absorber du liquide et à jouer le rôle d'éponge. La capacité d'absorption et la vitesse d'absorption sont les deux critères évaluant la qualité d'un papier absorbant. Les conditions de test permettant de mesurer ces critères sont définis par la norme ISO 12625-8. Il serait souhaitable de pouvoir améliorer au moins en partie certains aspects des dispositifs connus de mesure et de test de du temps d'immersion et de la capacité d'absorption d'un papier absorbant.

Des dispositifs de mesure de la capacité d'absorption d'un papier "tissue" sont décrits dans Christopher Stieger :

"Tissue Water Absorption Tester", 6 février 2015 (2015-02-06), XP055501096,Extrait de l'Internet: URL: https://xell.at/wp-content/uploads/2015/03/Data-Sheet-13.100-Tissue-Absorption-Tester.pdf,
Xell: "Xell Tissue Absorption Tester Digital Model",YouTube, 11 mai 2017 (2017-05-11), pages 1-1, XP054978609,Extrait de l'Internet: URL: https://www.youtube.com/watch?v=ryqsJbr-OM0 ou N Giede: "TISSUE ABSORPTION TESTER",, décembre 2014 (2014-12), XP055501111,Extrait de l'Internet:URL:http://www.mctec.nl/wp-content/uploads/2014/12/Water-Absorption-Tester.pdf

Résumé

Ainsi, un mode de réalisation prévoit un appareil de mesure du temps d'immersion et de la capacité d'absorption d'un échantillon de papier, comprenant, entre autres, un réceptacle apte à attraper l'échantillon dans un réservoir d'eau.

Selon un mode de réalisation, l'appareil de mesure comprend en outre un dispositif adapté à suspendre et à lâcher l'échantillon dans le réservoir d'eau.

Selon un mode de réalisation, le dispositif comprend un système d'accroche de l'échantillon et un moteur adapté à lâcher l'échantillon.

Selon un mode de réalisation, l'appareil de mesure comprend en outre un système de mesure adapté à mesurer le temps d'immersion de l'échantillon dans l'eau et le poids de l'eau absorbée par l'échantillon.

Selon un mode de réalisation, l'appareil de mesure comprend en outre un panier grillagé adapté à recevoir l'échantillon.

Selon un mode de réalisation, le panier a une masse comprise entre 2,9 g et 3,1 g.

Selon un mode de réalisation, le réservoir d'eau est disposé sur une plateforme réglable en hauteur.

Selon un mode de réalisation, l'appareil de mesure comprend en outre un système de commande adapté à commander le réglage en hauteur de la plateforme en fonction d'une mesure de poids.

Selon un mode de réalisation, le système de commande est adapté à déterminer le réglage en hauteur de la plateforme par rapport à la position du panier, en fonction du poids de l'eau absorbée par l'échantillon et de la forme du réservoir d'eau.

Selon un mode de réalisation, le système de commande est adapté à régler la hauteur de la plateforme pour que le panier soit à une première distance comprise entre 20,0 mm et 30,0 mm de la surface de l'eau du réservoir d'eau.

Selon un mode de réalisation, le réceptacle est une écumoire.

Un autre mode de réalisation prévoit un procédé de mesure du temps d'immersion et de la capacité d'absorption d'un échantillon de papier comprenant les étapes successives suivantes : suspendre l'échantillon au-dessus d'un réservoir d'eau ; lâcher l'échantillon ; et attraper l'échantillon avec un réceptacle positionné dans le réservoir d'eau.

Selon un mode de réalisation, l'échantillon est suspendu par un dispositif tel que décrit précédemment et la mesure du temps d'immersion est réalisée par un chronomètre.

Selon un mode de réalisation, le réceptacle est incliné par rapport à l'horizontale à sa sortie du réservoir d'eau.

Selon un mode de réalisation, le procédé de mesure utilise pour sa mise en oeuvre un appareil de mesure.

Selon un autre aspect, un mode de réalisation prévoit un appareil de mesure du temps d'immersion d'un échantillon de papier, l'appareil comprenant: une première pièce adaptée à suspendre, et à lâcher, un panier contenant l'échantillon de papier ; un réceptacle positionné pour recevoir le panier; et un système de pesée adapté à mesurer le poids de l'ensemble de la première pièce et du réceptacle.

Selon un mode de réalisation, l'appareil de mesure comprend en outre une plateforme réglable en hauteur par un moteur pour monter et descendre un réservoir d'eau.

Selon un mode de réalisation, l'appareil de mesure comprend en outre un processeur adapté à commander le moteur sur la base d'une mesure de poids fournie par le système de pesée.

Selon un mode de réalisation, le processeur est adapté à déterminer un déplacement de la plateforme par rapport au panier sur la base du poids de l'eau absorbé par l'échantillon de papier et la forme du réservoir d'eau.

Un autre mode de réalisation prévoit un procédé de mesure du temps d'immersion d'un échantillon de papier comprenant: suspendre, par une première pièce, un panier contenant l'échantillon de papier ; lâcher, par la première pièce, le panier pour qu'il tombe dans un réservoir d'eau ; recevoir, par un réceptacle positionné dans le réservoir d'eau, le panier ; et

peser, par un système de pesée, le poids de l'ensemble de la première pièce et du réceptacle.

Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :

la figure 1 est une vue schématique en perspective d'un mode de réalisation d'un appareil de mesure ;
la figure 2 est une vue en perspective d'un panier métallique de l'appareil de mesure de la figure 1 ;
la figure 3 est une vue en perspective d'une plateforme de l'appareil de mesure de la figure 1 ;
la figure 4 est un organigramme illustrant un mode de mise en oeuvre d'un procédé de mesure ; et
la figure 5 est un graphique illustrant l'évolution du poids d'un échantillon pendant la mise en oeuvre d'une mesure d'après le procédé de la figure 4.

Description détaillée

De mêmes éléments ont été désignés par de mêmes références dans les différentes figures. Par souci de clarté, seuls les éléments utiles à la compréhension des modes de réalisation décrits ont été représentés et sont détaillés.

Dans la description qui suit, lorsque l'on fait référence à des qualificatifs de position absolue, tels que le terme "haut", ou relative, tels que les termes "dessus", "inférieur", etc., ou à des qualificatifs d'orientation, tels que les termes "horizontal", "vertical", etc., il est fait référence à l'orientation des figures ou à un appareil de mesure dans une position normale d'utilisation. Sauf précision contraire, les expressions "sensiblement", et "de l'ordre de" signifient à 10 % près, de préférence à 5 % près.

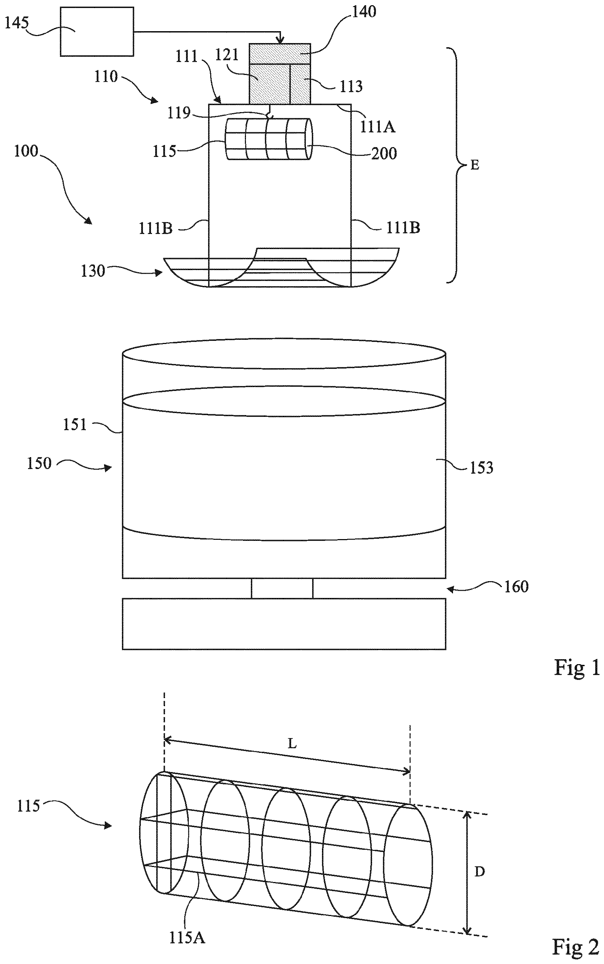

La figure 1 est une vue en perspective schématique d'un mode de réalisation d'un appareil 100 de mesure du temps d'immersion et de la capacité d'absorption d'un échantillon de papier absorbant 200. L'appareil 100 est, par exemple, apte à réaliser une mesure du temps d'immersion et de la capacité d'absorption de l'échantillon 200 conformément à la norme ISO 12625-8. Pour répondre à cette norme, l'échantillon 200 a une masse de 5 g ± 0,2 g, soit comprise entre 4,8 g et 5,2 g et une forme cylindrique à section circulaire. L'échantillon 200 est par exemple formé d'une feuille de papier absorbant enroulée sur elle-même pour prendre une forme cylindrique.

L'appareil 100 est équipé d'un dispositif 110 adapté à suspendre et à lâcher l'échantillon 200, d'un réceptacle 130, d'un système de mesure 140, d'un réservoir d'eau 150 et d'une plateforme 160.

Le dispositif 110 comprend un étrier articulé 111 comprenant un bras horizontal 111A et deux bras verticaux 111B ayant chacun une de ses extrémités reliée à une extrémité du bras horizontal 111A. Chaque bras vertical 111B est mobile par rapport au bras horizontal 111A. L'étrier 111 est combiné à un mécanisme 113 apte à l'incliner, et, plus particulièrement, apte à incliner le bras horizontal 111A d'un angle de 30 ° ± 1 ° par rapport à l'horizontale. Le bras 111A a une longueur comprise par exemple entre 90 mm et 150 mm. Les bras 111B ont une longueur comprise par exemple entre 120 mm et 200 mm. Le bras 111A est équipé d'un système d'accroche 119, piloté par un moteur 121. Le système 119 est apte à suspendre un panier ou une cage grillagée (par exemple métallique) 115. Le système 119 est par exemple un crochet et sera dénommé crochet dans la suite de la description. Le moteur 121 pilote l'ouverture et la fermeture du crochet 119, par exemple en pilotant l'inclinaison du crochet 119, pour permettre de lâcher le panier 115. Le panier 115 est adapté à recevoir l'échantillon 200 et sera décrit plus en détail en relation avec la figure 2. Dans une position de repos, le panier est sensiblement horizontal.

Le réceptacle 130, aussi dénommé écumoire, est positionné sous le panier 115 et est fixé au dispositif 110 par l'intermédiaire des bras 111B. L'écumoire 130 a des dimensions inférieures à celles du réservoir d'eau 150 afin de pouvoir être immergée totalement dans celui-ci, sans toucher les parois du réservoir d'eau 150. L'écumoire 130 est par exemple de forme semi-cylindrique et comprend des ouvertures permettant à de l'eau de s'évacuer. A titre d'exemple, l'écumoire 130 est grillagée.

Le système de mesure 140 comprend un système de pesée contrôlé, par exemple, par un système de commande 145, comprenant une mémoire, un circuit numérique, un chronomètre numérique, et/ou un ou plusieurs processeurs. Le système de pesée est adaptée à mesurer le poids d'un ensemble E comprenant le dispositif 110, l'échantillon 200 et l'écumoire 130 et à en déduire le poids de l'échantillon 200. Le poids du panier a par exemple été entré auparavant par un utilisateur et stocké dans la mémoire du système de commande 145. Dans un mode de réalisation, le système de pesée 140 mesure le poids de l'ensemble E avec une précision de 0,001 g, et le chronomètre numérique du système 145 est adapté à mesurer le temps d'immersion de l'échantillon dans le réservoir d'eau 150 avec une précision de 0,01 s.

Le réservoir d'eau 150 comprend une cuve 151 remplie d'eau 153 et positionnée sur la plateforme 160 (seule une portion de la plateforme 160 est représentée en figure 1). La cuve 151 est par exemple cylindrique à section circulaire ou à section rectangulaire. La cuve 151 est apte à contenir un volume minimal adapté à saturer en eau l'échantillon 200 pendant la mise en oeuvre du procédé de mesure. Ce volume d'eau est par exemple de l'ordre de 3,0 L. La cuve 151 a par exemple une hauteur comprise entre 120 mm et 200 mm. La cuve 151 est par exemple en verre ou en un plastique transparent. La cuve 151 peut être équipée d'un système de mise à niveau de l'eau, par exemple un tube incliné fermé par un bouchon amovible. L'eau 153 est par exemple de l'eau déminéralisée, de préférence de l'eau déminéralisée de conductivité inférieure à 0,25 mS/m. L'eau 153 est à une température comprise entre environ 22 et 24 °C, de préférence 23 °C.

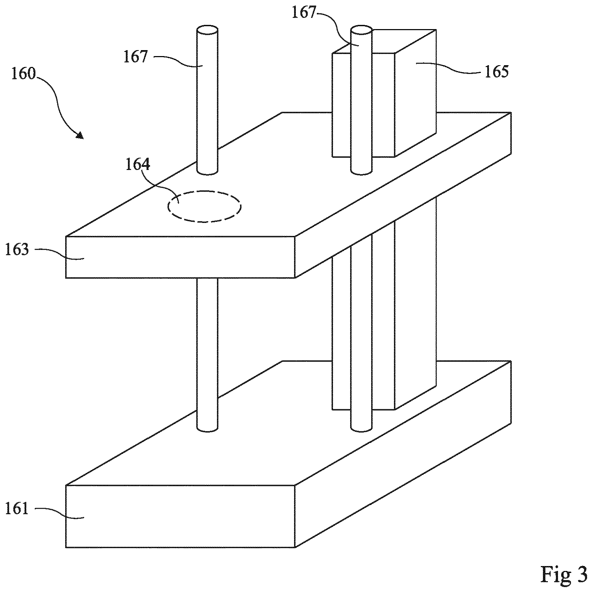

La plateforme 160 permet de déplacer verticalement la cuve 151 et sera décrite plus en détail en relation avec la figure 3.

La figure 2 est une vue schématique en perspective du panier 115 de l'appareil 100 de la figure 1. Le panier 115 est de forme cylindrique, adaptée à la forme de l'échantillon 200. Le panier 115 a une extrémité ouverte, permettant d'y glisser un échantillon 200, et une extrémité fermée. Le panier 115 est conçu et équilibré pour qu'une fois accroché au système d'accroche 119, il soit sensiblement horizontal.

Le panier 115 est grillagé. Autrement dit, il est formé à partir de fils métalliques 115A, par exemple en acier inoxydable. Le panier 115 a une fonction de protection (anti-écrasement) et de lest de l'échantillon 200. La densité du panier 115 permet à l'échantillon 200 d'éviter de flotter une fois qu'il est totalement immergé dans l'eau 153.

Afin de satisfaire la norme ISO 12625-8, le panier 115 a par exemple une masse de 3,0 g $\pm$ 0,1 g, soit comprise entre 2,9 g et 3,1 g, de préférence de 3,0 g. Les fils métalliques 115A ont un diamètre de l'ordre de 0,50 mm et une densité comprise entre 8,00 g/cm$^3$ et 8,10 g/cm$^3$, par exemple de l'ordre de 8,05 g/cm$^3$. Le panier 115 a une longueur L de 80,0 mm $\pm$ 1,0 mm, soit comprise entre 79,0 mm et 81,0 mm, de préférence de 80,0 mm, et un diamètre D de 50,0 mm $\pm$ 1,0 mm, soit compris entre 49,0 mm et 51,0 mm, de préférence de 50,0 mm. Les fils 115A sont assemblés en maille de forme carrée de coté de l'ordre de 20 mm.

La figure 3 est une vue schématique en perspective de la plateforme 160 selon un mode de réalisation. La plateforme 160 comprend un socle 161 et une nacelle 163. La nacelle 163 comprend un emplacement 164 pour le réservoir d'eau 150. La nacelle 163 est apte à se mouvoir verticalement par rapport au socle 161 grâce à un système de déplacement 165. Le système 165 comprend par exemple un moteur commandé par le système de commande 145 et une vis sans fin (ou un vérin) actionnée par le moteur. La trajectoire de la nacelle 163 est en outre stabilisée par l'intermédiaire de deux guides 167. Les guides 167 sont par exemple des tiges parallèles ayant chacune une extrémité fixée au socle 161 et guidant la nacelle 163 pendant un déplacement vertical. Plus particulièrement, la nacelle 163 comprend deux ouvertures par lesquelles les guides 167 la traversent.

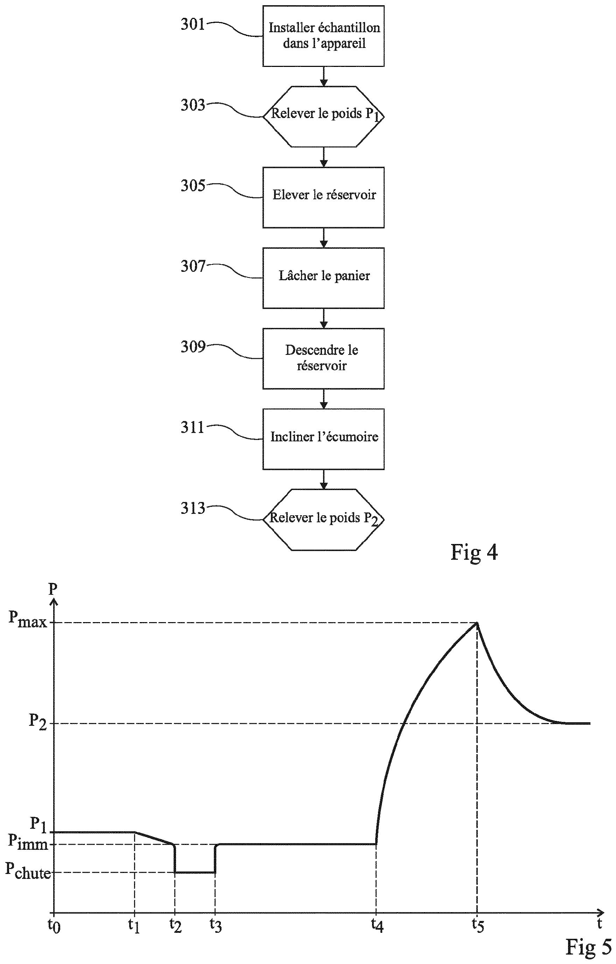

La figure 4 est un organigramme illustrant un mode de mise en oeuvre, par le system de commande 145, d'un procédé de mesure du temps d'immersion et de la capacité d'absorption de l'échantillon de papier absorbant 200 utilisant l'appareil de mesure 100 décrit en relation avec la figure 1. Avant toute mesure, on tare et on calibre par exemple le système de pesée du système de mesure 140. Pour cela, on met en oeuvre les étapes suivantes :

tarer le système de pesée en mesurant le poids du dispositif 110 et de l'écumoire 130 sans le panier ; et calibrer le système de pesée en mémorisant le poids du panier 115.

Pour cela le poids du panier 115 et son numéro de série sont par exemple entrés dans le système de commande 145 par un utilisateur par l'intermédiaire d'un clavier digital.

A une étape 301 ("Installer échantillon dans l'appareil"), l'échantillon de papier absorbant 200 est disposé dans le panier métallique 115. Ensuite, le panier 115 est accroché à la tige 111 par l'intermédiaire du crochet 119. Ainsi, l'appareil 100 est dans la position initiale illustrée en relation avec la figure 1. Plus particulièrement, l'échantillon 200, et le panier 115, sont dans une position horizontale au-dessus de l'écumoire 130.

A une étape 303 ("Relever le poids P1"), le système de mesure 140 réalise, via son système de pesée, une première mesure à sec du poids $P_1$ de l'échantillon 200 et du panier 115.

A une étape 305 ("Elever le réservoir"), le moteur de la plateforme 160 est actionné afin de déplacer verticalement la cuve 151. La cuve 151 est montée jusqu'à :

l'immersion totale de l'écumoire 130 dans l'eau 153 ; et
une situation dans laquelle le fond du panier 115 est à une distance par exemple de 25,0 mm $\pm$ 5,0 mm, soit comprise entre 20,0 mm et 30,0 mm, de préférence 25,0 mm de la surface de l'eau 153.

A une étape 307 ("Lâcher le panier") et après avoir attendu que la surface de l'eau 153 redevienne lisse, le moteur 121 actionne le crochet 119 afin de lâcher le panier 115 et l'échantillon 200 dans le réservoir d'eau. L'échantillon 200 absorbe de l'eau puis coule vers le fond de la cuve. Le panier 115 entre alors en contact avec l'écumoire 130. Le chronomètre du système de commande 145 mesure le temps d'immersion de l'échantillon 200, c'est-à-dire la durée pendant laquelle le panier 115 et l'échantillon 200 coule dans le cuve jusqu'à reposer sur l'écumoire 130. Cette mesure sera détaillée en relation avec la figure 5.

Ainsi, l'écumoire 130 est adapté à attraper ou récupérer l'échantillon 200, en d'autres termes, l'écumoire est positionnée dans la cuve de façon que l'échantillon 200, une fois tombé dans la cuve, soit supporté par l'écumoire en étant intégralement immergé.

A une étape 309 ("Descendre le réservoir"), on compte par exemple trente secondes et le moteur de la plateforme 160 est actionné afin de remettre le réservoir d'eau 150 dans son état initial. L'écumoire 130 est totalement émergée et le panier 115 et l'échantillon 200 repose dans l'écumoire.

A une étape 311 ("Incliner l'écumoire"), le mécanisme 113 est activé pour incliner l'étrier 111 d'un angle de l'ordre de 30 ° par rapport à l'horizontale. L'écumoire 130 s'incline alors d'un angle de l'ordre de 30 ° par rapport à l'horizontale. L'écumoire reste dans cette position pendant une durée de 60 s ± 1 s, soit comprise entre 59 et 61 s, de préférence 60 s, pour permettre à un surplus d'eau non absorbé par l'échantillon 200 d'être évacué.

A une étape 313 ("Relever le poids P2"), le système de mesure 140 réalise, via son système de pesée, une deuxième mesure du poids $P_2$ de l'échantillon 200 et du panier 115. Le poids $P_2$ est comparé au poids $P_1$ afin de déterminé le poids de l'eau absorbée. La capacité d'absorption de papier absorbant est égale au rapport entre la masse d'eau absorbée et la masse à sec du papier absorbant.

Pour que les mesures du temps d'immersion et de la capacité d'absorption soient conformes à la norme ISO 12625-8, l'eau 153 est renouvelée après 5 procédés de mesure ou essais. L'appareil 100 est de plus équipé d'un logiciel embarqué qui signale à l'utilisateur que l'eau peut être changée après 10 essais. Le moteur de la plateforme est de plus équipé d'un système d'asservissement, par exemple contrôlé par le système de commande 145, permettant d'ajuster la hauteur de la plateforme essai après essai. Plus particulièrement, le système de commande 145 effectue un calcul d'asservissement, qui prend en compte la diminution du volume d'eau 153 dans la cuve 151 pendant chaque essai. Cette diminution du volume est calculée par le système de commande 145 à partir de la masse d'eau absorbée par l'échantillon 200 pendant l'essai. La différence de hauteur d'eau Δh est ensuite calculée avec la relation suivante :

$$\Delta h = \frac{V_{abs}}{S_{cuve}},$$

dans laquelle $V_{abs}$ est le volume d'eau absorbée et $S_{cuve}$ est la surface de l'eau 153 dans la cuve 151. Pendant l'étape 305, le système de commande 145 recalcule la position de la cuve 151 en tenant compte de la hauteur d'eau Δh manquante dans la cuve 151, et commande le système d'asservissement en conséquence de façon que la cuve 151 soit toujours disposée à une distance de l'ordre de 25,0 mm du fond du panier 115.

La figure 5 est un graphique illustrant l'évolution du poids P de l'ensemble E comprenant le dispositif 110, l'échantillon 200 et l'écumoire 130 pendant la mise en oeuvre du procédé de mesure décrit en relation avec la figure 4.

Avant un instant $t_0$, l'échantillon 200 est installé dans l'appareil 100 (étape 301).

Entre l'instant $t_0$ et un instant $t_1$, on mesure le poids $P_1$ (étape 303). Le poids P reste constant au niveau $P_1$ pendant ces deux étapes.

Entre l'instant $t_1$ et un instant $t_2$, la plateforme est déplacée verticalement. La cuve 151 se rapproche de l'écumoire 130 jusqu'à l'immerger complètement et positionner le fond du panier 115 à une distance de l'ordre de 25 mm de la surface de l'eau (étape 305). Le poids P décroit du fait de l'apparition d'une poussée d'Archimède s'appliquant sur l'écumoire 130, le temps que l'échantillon soit immergé.

A l'instant $t_2$, le moteur 121 actionne le crochet 119 qui lâche le panier 115 et l'échantillon 200 (étape 307). Le poids $P_{chute}$ mesuré pendant la chute du panier 115 et de l'échantillon 200 représente le poids stabilisé de l'ensemble E immergé, sans le panier 115 et l'échantillon 200, et est inférieur au poids $P_1$. Pendant la chute du panier 115 et de l'échantillon 200 dans l'eau 153, le système de pesée enregistre une réduction du poids par exemple de l'ordre de 8 g correspondant au poids du panier et de l'échantillon.

A l'instant $t_3$, le panier 115 et l'échantillon 200 reposent sur l'écumoire 130.

Un premier temps d'immersion de l'échantillon 200, mesuré par le chronomètre du système de mesure 145, correspond par exemple à la durée de la chute de l'échantillon jusqu'à son contact avec l'écumoire 130. La mesure de cette durée est effectuée à l'aide de la mesure du poids P pendant le procédé de mesure, soit la durée séparant $t_2$ et $t_3$. L'instant $t_2$ est mesuré au moment où le moteur 121 actionne le crochet 119. A cet instant, le poids de l'ensemble E décroit d'une valeur par exemple de l'ordre de 8 g. L'instant t3 est mesuré au moment où le poids P augmente d'une valeur seuil de l'ordre de quelques grammes lorsque le panier 115 entre en contact avec l'écumoire 130. La longueur des bras 111B est adaptée pour positionner le fond de l'écumoire 130 à une distance de la surface de l'eau 153 égale au diamètre du panier, et d'environ 50 mm dans cet exemple.

Entre l'instant $t_3$ et un instant $t_4$, on compte un temps de mouillage de par exemple trente secondes pendant que l'échantillon 200 est immergé dans l'eau 153. Le poids P se stabilise à une valeur $P_{imm}$. L'intervalle de temps entre les instants $t_3$ et $t_4$ permet la saturation en eau de l'échantillon 200 avant l'instant $t_4$.

A l'instant $t_4$, la plateforme 160 est abaissée verticalement et le panier 115 et l'échantillon 200 sont émergés de l'eau 153 (étape 309). Ainsi, le poids P de l'ensemble E augmente jusqu'à un poids maximal $P_{max}$

atteint à un instant $t_5$. A l'instant $t_5$, l'écumoire 130 est inclinée pour évacuer le surplus d'eau non absorbée par l'échantillon contenu entre les plis de l'échantillon (étape 311). Le poids P se stabilise à un poids $P_2$.

Le relevé du poids $P_2$ est effectué à un instant postérieur à l'instant $t_5$, qui peut être de par exemple soixante secondes après l'instant $t_4$ afin que le poids de l'ensemble E soit stabilisé.

Des modes de réalisation particuliers ont été décrits. Diverses variantes et modifications apparaîtront à l'homme de l'art. En particulier, l'écumoire 130 pourrait se déplacer par rapport à la cuve 151.

De plus, bien que diverses plages de valeurs et un seul procédé de mesure ont été mentionnés afin de conformer la mesure à la norme 12625-8, d'autres valeurs et d'autres procédés de mesure pourront être envisagés.

**Revendications**

1. Appareil (100) de mesure du temps d'immersion et de la capacité d'absorption d'un échantillon de papier (200), comprenant, entre autres, un réceptacle (130) apte à attraper l'échantillon (200) dans un réservoir d'eau (150), et un système de mesure du temps d'immersion et de la capacité d'absorption de l'échantillon.

2. Appareil selon la revendication 1, comprenant en outre un dispositif (110) adapté à suspendre et à lâcher l'échantillon (200) dans le réservoir d'eau (150).

3. Appareil selon la revendication 1 ou 2, dans lequel le dispositif (110) comprend un système d'accroche (119) de l'échantillon (200) et un moteur adapté à lâcher l'échantillon (200).

4. Appareil selon l'une quelconque des revendications 1 à 3, dans lequel le système de mesure (140) est adapté à mesurer le temps d'immersion de l'échantillon (200) dans l'eau et le poids de l'eau absorbée par l'échantillon (200).

5. Appareil selon l'une quelconque des revendications 1 à 4, comprenant en outre un panier grillagé (115) adapté à recevoir l'échantillon (200).

6. Appareil selon la revendication 5, dans lequel le panier (115) a une masse comprise entre 2,9 g et 3,1 g.

7. Appareil selon l'une quelconque des revendications 1 à 6, dans lequel le réservoir d'eau (150) est disposé sur une plateforme (160) réglable en hauteur.

8. Appareil selon la revendication 7, comprenant en outre un système de commande (145) adapté à commander le réglage en hauteur de la plateforme (160) en fonction d'une mesure de poids.

9. Appareil selon la revendication 8, dans lequel le système de commande (145) est adapté à déterminer le réglage en hauteur de la plateforme (160) par rapport à la position du panier (115), en fonction du poids de l'eau absorbée par l'échantillon (200) et de la forme du réservoir d'eau (150) pendant une mesure précédente.

10. Appareil selon la revendication 8 ou 9, dans lequel le système de commande (145) est adapté à régler la hauteur de la plateforme (160) pour que le panier (115) soit à une première distance comprise entre 20,0 mm et 30,0 mm de la surface de l'eau du réservoir d'eau (150).

11. Appareil selon l'une quelconque des revendications 1 à 10, dans lequel le réceptacle (130) est une écumoire.

12. Procédé de mesure du temps d'immersion et de la capacité d'absorption d'un échantillon (200) de papier comprenant les étapes successives suivantes :

   suspendre (301) l'échantillon (200) au-dessus d'un réservoir d'eau (150) ;
   lâcher (307) l'échantillon (200) ;
   attraper l'échantillon (200) avec un réceptacle (130) positionné dans le réservoir d'eau (150) ; et
   mesurer le temps d'immersion et la capacité d'absorption de l'échantillon (200).

13. Procédé selon la revendication 12, dans lequel l'échantillon est suspendu par un dispositif (110) et la mesure du temps d'immersion est réalisé par un chronomètre.

14. Procédé selon la revendication 12 ou 13, dans lequel le réceptacle (130) est incliné (311) par rapport à l'horizontale à sa sortie du réservoir d'eau (150).

15. Procédé selon l'une quelconque des revendications 12 à 14, utilisant pour sa mise en oeuvre un appareil (100) de mesure selon l'une quelconque des revendications 1 à 11.

**Patentansprüche**

1. Vorrichtung (100) zur Messung der Eintauchzeit und der Absorptionskapazität einer Papierprobe (200), wobei die Vorrichtung (100) unter anderem einen Behälter (130), der die Probe (200) in einem Wasserbehälter (150) auffangen kann, und ein System zur Messung der Eintauchzeit und der Absorptionskapazität der Probe aufweist.

2. Vorrichtung nach Anspruch 1, die ferner eine Einrichtung (110) aufweist, die in der Lage ist, die Probe (200) aufzuhängen und in den Wasserbehälter (150) fallenzulassen.

3. Vorrichtung nach Anspruch 1 oder 2, wobei die Einrichtung (110) ein System (119) zum Aufhängen der Probe (200) und einen Motor, der die Probe (200) fallenlassen kann, aufweist.

4. Vorrichtung nach einem der Ansprüche 1 bis 3, wobei das Messsystem (140) in der Lage ist, die Eintauchzeit der Probe (200) in das Wasser und das Gewicht des von der Probe (200) absorbierten Wassers zu messen.

5. Vorrichtung nach einem der Ansprüche 1 bis 4, die ferner einen Gitterkorb (115) aufweist, der die Probe (200) aufnehmen kann.

6. Vorrichtung nach Anspruch 5, wobei der Korb (115) eine Masse im Bereich von 2,9 g bis 3,1 g hat.

7. Vorrichtung nach einem der Ansprüche 1 bis 6, wobei der Wasserbehälter (150) auf einer höhenverstellbaren Plattform (160) angeordnet ist.

8. Vorrichtung nach Anspruch 7, die ferner ein Steuersystem (145) aufweist, das in der Lage ist, die Höheneinstellung der Plattform (160) entsprechend einer Gewichtsmessung zu steuern.

9. Vorrichtung nach Anspruch 8, wobei das Steuersystem (145) in der Lage ist, die Höheneinstellung der Plattform (160) in Bezug auf die Position des Korbes (115) entsprechend dem Gewicht des von der Probe (200) absorbierten Wassers und der Form des Wasserbehälters (150) während einer vorherigen Messung zu bestimmen.

10. Vorrichtung nach Anspruch 8 oder 9, wobei das Steuersystem (145) in der Lage ist, die Höhe der Plattform (160) so einzustellen, dass sich der Korb (115) in einem ersten Abstand im Bereich von 20,0 mm bis 30,0 mm von der Wasseroberfläche des Wasserbehälters (150) befindet.

11. Vorrichtung nach einem der Ansprüche 1 bis 10, wobei der Behälter (130) ein Skimmer ist.

12. Ein Verfahren zur Messung der Eintauchzeit und der Absorptionskapazität einer Papierprobe (200), das die folgenden aufeinanderfolgenden Schritte aufweist:

Aufhängen (301) der Probe (200) über einem Wasserbehälter (150);
Fallenlassen (307) der Probe (200);
Auffangen der Probe (200) mit einem in dem Wasserbehälter (150) angeordneten Behälter (130); und
Messen der Eintauchzeit und der Absorptionskapazität der Probe (200).

13. Verfahren nach Anspruch 12, bei dem die Probe durch eine Einrichtung (110) aufgehängt wird und die Eintauchzeit durch einen Chronometer gemessen wird.

14. Verfahren nach Anspruch 12 oder 13, bei dem der Behälter (130) bei der Entnahme aus dem Wasserbehälter (150) gegenüber der horizontalen Richtung geneigt (311) wird.

15. Verfahren nach einem der Ansprüche 12 bis 14, bei dem für seine Durchführung die Messvorrichtung (100) nach einem der Ansprüche 1 bis 11 verwendet wird.

**Claims**

1. A device (100) for measuring the immersion time and the absorption capacity of a paper sample (200), comprising, among others, a receptacle (130) capable of catching the sample (200) in a water tank (150), and a system for measuring the immersion time and the absorption capacity of the sample.

2. The device according to claim 1, further comprising a device (110) capable of suspending and of dropping the sample (200) into the water tank (150).

3. The device according to claim 1 or 2, wherein the device (110) comprises a system (119) for hanging the sample (200) and a motor capable of dropping the sample (200).

4. The device according to any of claims 1 to 3, wherein the measurement system (140) is capable of measuring the time of immersion of the sample (200) in the water and the weight of the water absorbed by the sample (200).

5. The device according to any of claims 1 to 4, further comprising a wire basket (115) capable of receiving the sample (200).

6. The device according to claim 5, wherein the basket (115) has a mass in the range from 2.9 g to 3.1 g.

7. The device according to any of claims 1 to 6, wherein the water tank (150) is arranged on a platform (160) of adjustable height.

8. The device according to claim 7, further comprising a control system (145) capable of controlling the height adjustment of the platform (160) according to a weight measurement.

9. The device according to claim 8, wherein the control system (145) is capable of determining the height adjustment of the platform (160) with respect to the position of the basket (115), according to the weight of the water absorbed by the sample (200) and to the shape of the water tank (150) during a previous measurement.

10. The device according to claim 8 or 9, wherein the control system (145) is capable of adjusting the height of the platform (160) so that the basket (115) is at a first distance in the range from 20.0 mm to 30.0 mm from the surface of the water of the water tank (150).

11. The device according to any of claims 1 to 10, wherein the receptacle (130) is a skimmer.

12. A method of measuring the immersion time and the absorption capacity of a paper sample (200) comprising the successive steps of:

  suspending (301) the sample (200) above a water tank (150) ;
  dropping (307) the sample (200);
  catching the sample (200) with a receptacle (130) positioned in the water tank (150); and
  measuring the immersion time and the absorption capacity of the sample (200).

13. The method according to claim 12, wherein the sample is suspended by a device (110) and the immersion time is measured by a chronometer.

14. The method according to claim 12 or 13, wherein the receptacle (130) is inclined (311) with respect to the horizontal direction as it is taken out of the water tank (150).

15. The method according to any of claims 12 to 14, using for its implementation the measurement device (100) according to any of claims 1 to 11.

Fig 1

Fig 2

Fig 3

301 — Installer échantillon dans l'appareil

303 — Relever le poids $P_1$

305 — Elever le réservoir

307 — Lâcher le panier

309 — Descendre le réservoir

311 — Incliner l'écumoire

313 — Relever le poids $P_2$

Fig 4

Fig 5

# EP 3 470 836 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- *Tissue Water Absorption Tester,* 06 Février 2015, https://xell.at/wp-content/uploads/2015/03/Data-Sheet-13.100-Tissue-Absorption-Tester.pdf

- **XELL.** *Xell Tissue Absorption Tester Digital Model,* 11 Mai 2017, 1-1, https://www.youtube.com/watch?v=ryqsJbr-OM0
- **N GIEDE.** *TISSUE ABSORPTION TESTER,* Décembre 2014, http://www.mctec.nl/wp-content/uploads/2014/12/Water-Absorption-Tester.pdf